# EUROPEAN PATENT SPECIFICATION

(11) **EP 3 650 442 B1**
(45) Date of publication and mention of the grant of the patent: **30.06.2021**
(21) Application number: 18205535.0
(22) Date of filing: 09.11.2018
(51) Int. Cl.: C07D 231/14

(54) **METHOD FOR THE PREPARATION OF DIFLUOROMETHYL PYRAZOLE CARBOXYLIC ALKYL ESTER AND ITS ACID**
VERFAHREN ZUR HERSTELLUNG VON DIFLUORMETHYL-PYRAZOL-CARBONSÄURE-ALKYLESTER UND DESSEN SÄURE
PROCÉDÉ DE PRÉPARATION DE D'ESTER D'ALKYLE D'ACIDE CARBOXYLIQUE DE PYRAZOLE DIFLUOROMÉTHYL ET SON ACIDE

(43) Date of publication of application: 13.05.2020
(73) Proprietor: Lonza Solutions AG, 3930 Visp (CH)
(72) Inventor: TAESCHLER, Christoph, 3930 Visp (CH); NAKAJIMA, Masaki, 3600 Thun (CH); BREUER, Andreas, 3905 Saas-Almagell (CH); BIEHL, David, 3930 Visp (CH); BELSER, Thomas, 3902 Glis (CH)
(74) Representative: Greiner, Elisabeth

(56) References cited:
- WO-A1-2005/123690
- WO-A2-2008/152138
- US-A- 5 093 347

## Description

Subject of the invention is a method for the preparation of difluoromethyl pyrazole carboxylic alkyl ester and of difluoromethyl pyrazole carboxylic acid by a reaction of 2,2-difluoroactetate alkyl ester with 3,3-dialkoxypropanoate alkyl ester, cyclization of the reaction product with methyl hydrazine providing difluoromethyl pyrazole carboxylic alkyl ester and optionally saponification of the obtained difluoromethyl pyrazole carboxylic alkyl ester.

### BACKGROUND OF THE INVENTION

Difluoromethyl pyrazole carboxylic acid is used as an intermediate in the preparation of fungicides which are used for control of agricultural diseases.

US 5,093,347 discloses in example 1 the reaction of ethyl difluoroacetoacetate with triethylorthoformate providing ethyl 2-ethoxymethylene-4, 4-difluoro-3-oxo butyrate and its subsequent cyclization with methyl hydrazine to provide ethyl 3-difluoromethyl-1-methyl-4-pyrazole carboxylate, which then is saponified to provide the acid.
The yield of the first two steps providing the ester is 63%.

There was a need for an improved process having high yield and having a low content of the respective unwanted isomer isoDFP-ester and iso-DFPA.

A method was found that provides the ester in two steps with a yield of over 80%, additionally the unwanted isomer occurs only in low amounts.

### Abbreviations:

- EDFA: ethyl 2,2-difluoroacetate, compound of formula (7)
- DF-DMPNa: (2-(dimethoxy-methyl)-4,4-difluoro-1-methoxy-1,3-dioxobutan-2-yl) sodium salt, compound of formula (5-Na)
- DF-DMPK: (2-(dimethoxy-methyl)-4,4-difluoro-1-methoxy-1,3-dioxobutan-2-yl) potassium salt, compound of formula (5-K)
- DFPA: 3-(difluoromethyl)-1-methyl-1H-pyrazo le-4-carboxylic acid, compound of formula (3)
- DFP-ester: methyl 3-(difluoromethyl)-1-methyl-1H-pyrazole-4-carboxylate, compound of formula (1)
- eq: equivalent, usually molar equivalents, if the stated otherwise
- iso-DFPA: 5-(difluoromethyl)-1-methyl-1H-pyrazole-4-carboxylic acid, compound of formula (4)

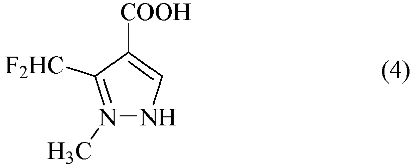

- isoDFP-ester: methyl 5-(difluoromethyl)-1-methyl-1H-pyrazole-4-carboxylate, compound of formula (2) or compound of formula (II) with the alkyl chain of the ester residue being R1, with R1 as defined herein

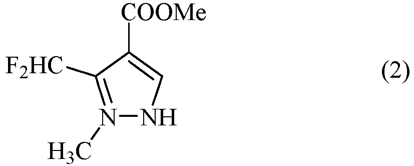

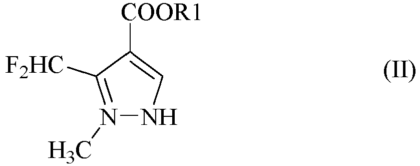

- MDMP: methyl 3,3-dimethoxypropanoate, compound of formula (6)
- MeH: methyl hydrazine
- MeTHF: methyltetrahydrofuran, usually 2-MeTHF, i.e. 2-methyltetrahydrofuran
- MTBE: methyl-tert-butylether
- THF: tetrahydrofuran
- %: usually weight percent (wt%), except for the yield, which is mol%, if not stated otherwise

### SUMMARY OF THE INVENTION

Subject of the invention is a method for the preparation of compound of formula (I); 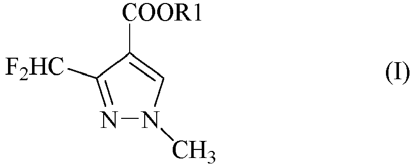 the method comprises two steps, a step STEP1 and a step STEP2;
STEP1 comprises a reaction REAC1 of compound of formula (VII) and compound of formula (VI) 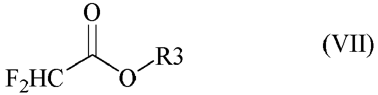 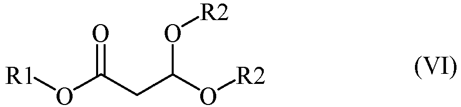 in the presence of a base BAS1, BAS1 is compound of formula (VIII); 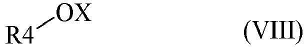
R1, R2, R3 and R4 are identical or different and independently from each other C₁₋₄ alkyl;
X is Na or K;
STEP2 comprises a reaction REAC2 of the reaction product of REAC1 with methyl hydrazine in the presence of an acid ACID2;
ACID2 is selected from the group consisting of AcOH, H₂SO₄, HCOOH, citric acid, benzoic acid, lactic acid and acidic ion exchange resin.

### DETAILED DESCRIPTION OF THE INVENTION

Preferably, R1, R2, R3 and R4 are identical or different and independently from each other selected from the group consisting of methyl, ethyl, propyl, and butyl;
more preferably methyl, ethyl, isopropyl, and tert butyl;
even more preferably methyl, ethyl, and tert butyl.

In an embodiment,
R1 is preferably methyl or ethyl, more preferably methyl;
R2 is preferably methyl or ethyl, more preferably methyl;
R3 is preferably methyl or ethyl, more preferably ethyl;
R4 is preferably ethyl or tert butyl, more preferably tert butyl.

Preferably, R1 and R2 are identical.

Preferably, X is Na.

In another embodiment,
BAS1 is preferably NaOEt, NaOtBu or KOtBu;
more preferably NaOEt or NaOtBu.

Preferably, compound of formula (I) is compound of formula (1); 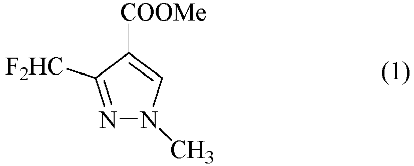 compound of formula (VII) is compound of formula (7); and 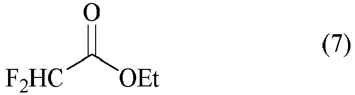 compound of formula (VI) is compound of formula (6);

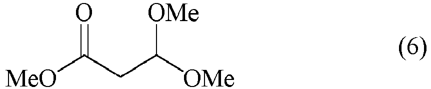

Preferably, the reaction product of REAC1 is used for STEP2 in form of the reaction mixture obtained from REAC1.

The reaction product of REAC1 is believed to be compound of formula (V); 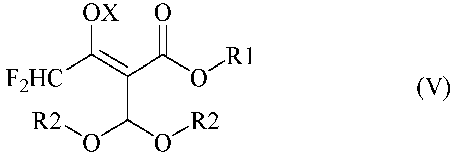
with R1 and R2 as defined herein, also with all their embodiments;
embodiments of compound of formula (V) are compound of formula (5-Na) and compound of formula (5-K), when compound of formula (VII) is compound of formula (7) and when compound of formula (VI) is compound of formula (6).

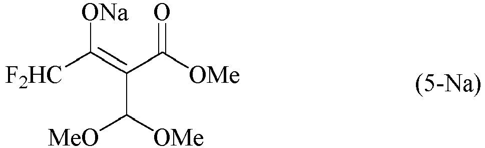

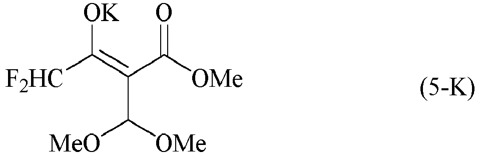

Depending on the combination of R1, R2 and R3 in compound of formula (VII) and compound of formula (VI) on one side with R4 in compound of formula (VIII) on the other side, the reaction product of STEP 1 or of STEP2 or of both steps can be a mixture due to an exchange of the alkoxide from BAS1 with an alkoxide residue in any the ester groups of compound of formula (VII) and compound of formula (VI) or in the dialkoxymethyl group of compound of formula (V); this possibility is known to the skilled person.

Preferably, the molar amount of compound of formula (VI) in REAC1 is from 1 to 5 eq, more preferably from 1.05 to 4 eq, even more preferably from 1.05 to 3 eq, especially from 1.1 to 2.5 eq, of the molar amount of compound of formula (VII).

Preferably, the molar amount of BAS1 in REAC1 is from 1 to 2 eq, more preferably from 1 to 1.75 eq, even more preferably from 1 to 1.5 eq, especially from 1 to 1.25 eq, more especially from 1 to 1.15 eq, of the molar amount of compound of formula (VII).

REAC1 can be done neat or in the presence of a solvent SOLV1;
preferably, SOLV1 is selected from the group consisting of methanol, ethanol, n-propanol, isopropanol, n-butanol, iso-butanol, sec-butanol, tert-butanol, THF, 2-MeTHF and MTBE, toluene, n-butyl acetate, and diisopropylether;
more preferably, SOLV1 is selected from the group consisting of methanol, THF, 2-MeTHF and MTBE.

Preferably the amount of a SOLV1 is from 1 to 10 fold, more preferably from 1 to 5 fold, of the weight of compound of formula (VII).

BAS1 can be used for REAC1 as substance without a solvent, or it can be used in form of a solution of suspension in a solvent such as the alcohol from which BAS1 is derived, or SOLV1, preferably in SOLV1.

Preferably, for REAC1 a mixture of compound of formula (VI) with compound of formula (VII) is added to BAS1 or vice versa.

Preferably, REAC1 is done at a temperature TEMPREAC1, TEMPREAC1 is from 0 to 70°C, more preferably from 5 to 70 °C, even more preferably of from 10 to 67.5 °C.

Preferably, the reaction time TIMEREAC1 of REAC1 is from 10 min to 12 h, more preferably of from 45 min to 8 h, even more preferably of from 45 min to 7 h.

Preferably, REAC1 is done for at least a TIMEREAC1 of from 1 to 6 h at a TEMPREAC1 of from 50 to 67.5°C.

Preferably, REAC1 can be done at atmospheric pressure or at elevated pressure, depending on the chosen target TEMPREAC1 and the vapour pressure of the reaction mixture at this chosen target TEMPREAC1.

Preferably, the acidic ion exchange resin is a polymeric sulfonic acid resin.

The polymeric sulfonic acid resin is preferably an acidic cation exchange resin, more preferably a weakly or a strongly acidic cation exchange resin, for example such as used in heterogeneous acid catalysis.

Preferably, the polymeric sulfonic acid resin has an average molecular weight of from 1000 to 1000000 D; and/or
preferably, a concentration of acid sites of from 1 to 15, more preferably of from 1 to 11.6, even more preferably of from 1 to 10, especially of from 1 to 8, more especially of from 1 to 7 equivalents per kg resin; and/or
preferably, an acid number of from 1 to 650, more preferably of from 1 to 560, even more preferably of from 1 to 450, especially of from 1 to 350, more especially of from 50 to 650, even more especially of from 1 to 560, in particular of from 50 to 450, more in particular of from 50 to 350; and/or
preferably, a particle size of from 4 to 800 mesh, more preferably 4 to 400 mesh.

The concentration of acid sites is determined by the Master Test Method MTM 0232, Edition 1.4, ©Rohm and Haas Company, 1998, wherein the CATALYST VOLATILES are determined by the Master Test Method MTM 0126, Edition 1.6, ©Rohm and Haas Company, 2000.

The acid number is determined according to DIN EN ISO 3682. For further explanation of the acid number and for its relation to the concentration of acid sites see "BASF Handbuch Lackiertechnik", Artur Goldschmidt and Hans-Joachim Streitberger, Vincentz Verlag, 2002, ISBN 3-87870-324-4, chapter 2.3.2.2 (pages 272 to 273). According to the teaching therein, an concentration of acid sites of 1 equivalents per kg equals an acid number of 56, therefore a concentration of acid sites of 4.7 equivalents per kg equals an acid number of 263.

Especially, the polymeric sulfonic acid resin is selected from the group consisting of sulfonated polystyrene resins, sulfonated polystyrene resins cross linked with divinyl benzene and poly(2-acrylamido-2-methyl-1-propanesulfonic acid).

Sulfonated polystyrene resins cross linked with divinyl benzene are also called divinylbenzene-styrenesulfonic acid copolymer.

Examples for a polymeric sulfonic acid resin is Amberlyst® 15 DRY, Amberlite CG50 and Dowex Mac-3.

Preferably, ACID2 is selected from the group consisting of AcOH, H₂SO₄, HCOOH, citric acid, benzoic acid, and lactic acid;
more preferably, ACID2 is AcOH or H₂SO₄;
even more preferably, ACID2 is AcOH.

Methyl hydrazine in REAC2 is preferably used in aqueous form; more preferably as aqueous methyl hydrazine 30 to 45 wt%, even more preferably as aqueous methyl hydrazine 40 to 45 wt%.

Preferably, the molar amount of methyl hydrazine in REAC2 is from 1 to 1.2 eq, more preferably from 1 to 1.1 eq, even more preferably from 1 to 1.05 eq, especially from 1 to 1.01 eq, of the molar amount of compound of formula (VII).

ACID2 in REAC2 can used neat or as aqueous solution.

Preferably, the molar amount of ACID2 in REAC2 is at least 1 eq, of the molar amount of compound of formula (VII), the eq being base in the molar equivalents of acid hydrogen atoms in ACID2.

Preferably, the molar amount of ACID2 in REAC2 is from 1 to 2 eq, more preferably from 1 to 1.75 eq, even more preferably from 1 to 1.5 eq, of the molar amount of compound of formula (VII), the eq being base in the molar equivalents of acid hydrogen atoms in ACID2.

REAC2 can be done neat or in the presence of a solvent SOLV2;
Preferably, SOLV2 is selected from the group consisting of methanol, ethanol, n-propanol, isopropanol, n-butanol, iso-butanol, sec-butanol, tert-butanol, THF, 2-MeTHF, MTBE, toluene, n-butyl acetate, and diisopropyl ether;
more preferably, SOLV2 is selected from the group consisting of methanol, THF, 2-MeTHF, MTBE, toluene, n-butyl acetate, methanol, diisopropylether;
even more preferably, SOLV2 is selected from the group consisting of methanol, 2-MeTHF, MTBE, toluene, n-butyl acetate, methanol, diisopropylether.

Preferably the amount of a SOLV2 is from 1 to 10 fold, more preferably from 1 to 5 fold, even more preferably from 1 to 3 fold, especially from 1.5 to 3 fold of the weight of compound of formula (VII).

Preferably, the reaction mixture obtained from REAC1 is added to a mixture of ACID2 with methyl hydrazine. SOLV1 can for example be added to the mixture of ACID2 with methylhydrazine, to the reaction mixture obtained from REAC1, or to both.

Preferably, REAC2 is done in the presence of water.

Preferably, REAC2 is done at a temperature TEMPREAC2, TEMPREAC2 is from 0 to 70°C.

Preferably, the reaction time TIMEREAC2 of REAC2 is from 30 min to 12 h, more preferably of from 30 min to 8 h, even more preferably of from 30 min to 6 h, especially from 30 min to 4 h.

Preferably, REAC2 is done for at least a TIMEREAC2 of from 45 min to 3 h at a TEMPREAC2 of from 50 to 67.5°C.

Preferably, REAC2 can be done at atmospheric pressure or at elevated pressure, depending on the chosen target TEMPREAC2 and the vapour pressure of the reaction mixture at this chosen target TEMPREAC2.

Preferably STEP2 is done after STEP1 without isolation of the reaction product of REAC1.

After STEP2, compound of formula (I) can be isolated by standard methods known to the skilled person, such as evaporation of any solvent or water, precipitation, for example by addition of an anti solvent, filtration, washing and drying.

After STEP2, compound of formula (I), obtained from REAC2, can be saponified to provide the free acid.

Therefore another subject of the invention is a method for the preparation of compound of formula (3) or the sodium or potassium salt of compound of formula (3); 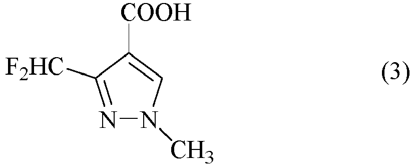
the method comprises the two steps STEP1, STEP2 and a third step STEP3;
with STEP1 and STEP2 as described herein, also with all their embodiments;
in STEP3 compound of formula (I) obtained from STEP2 is saponified.

The saponification of compound of formula (I) can be done under acidic, providing compound of formula (3), or under basic conditions, providing the salt of compound of formula (3).

Preferably, compound of formula (I) is saponified under basic conditions;
more preferably, compound of formula (I) is saponified with a base BAS3, BAS3 is NaOH or KOH;
preferably, BAS3 is NaOH.

Preferably, the molar amount of BAS3 in STEP3 is from 1 to 3 eq, more preferably from 1 to 2.5 eq, even more preferably from 1 to 2 eq, of the molar amount of compound of formula (VII).

Preferably, the saponification in STEP3 is done in the presence of water.

Preferably, the saponification in STEP3 is done at a temperature TEMPREAC3, TEMPREAC3 is from 0 to 70°C,more preferably from 20 to 70°C, even more preferably from 40 to 70°C, especially from 45 to 70°C.

Preferably, the reaction time TIMEREAC3 of the saponification in STEP3 is from 30 min to 12 h, more preferably of from 1 to 8 h, even more preferably of from 2 to 7 h.

Preferably, the saponification in STEP3 can be done at atmospheric pressure or at elevated pressure, depending on the chosen target TEMPREAC3 and the vapor pressure of the reaction mixture at this chosen target TEMPREAC3.

When the saponification is done under basic conditions, then after the saponification compound of formula (3) can be obtained by acidifying the reaction mixture after the saponification by addition of an ACID4, ACID4 is preferably H₂SO₄ or HCl.

Preferably, the molar amount of ACID4 is from 1 to 5 eq, more preferably from 1 to 4 eq, even more preferably from 1 to 3 eq, of the molar amount of compound of formula (VII).

Preferably, the acidification of the reaction mixture from STEP3 for obtaining compound of formula (3) is done at a temperature TEMPREAC4, TEMPREAC4 is from 0 to 70°C, more preferably from 20 to 70°C, even more preferably from 40 to 70°C, especially from 45 to 70°C.

Preferably, the time TIMEREAC4 of the acidification of the reaction mixture from STEP3 for obtaining compound of formula (3) is from 10 min to 1 h.

Preferably, the acidification of the reaction mixture from STEP3 for obtaining compound of formula (3) can be done at atmospheric pressure or at elevated pressure, depending on the chosen target TEMPREAC4 and the vapour pressure of the reaction mixture from STEP3 at this chosen target TEMPREAC4.

Preferably, the reaction mixture obtained from STEP2 is used for STEP3 without isolation of compound of formula (I) after STEP2.

After STEP3, compound of formula (3) can be isolated by standard methods known to the skilled person, such as acidification of the reaction mixture obtained from STEP3, evaporation of any solvent or water, precipitation, for example by addition of an anti solvent, filtration or by said acidification, washing and drying.

Compound of formula (VI) is a known compound and is preferably prepared as described in WO 2009/056293 A1 with R1 and R2 being identical.

So preferably, compound of formula (VI) is prepared by a continuous process for preparing alkyl 3,3-dialkoxypropionates of formula (R1-O)₂CHCH₂CO₂R1, wherein R1 is defined as herein, also with all its embodiments, by reacting ketene (CH₂=C=O) with an orthoformate of formula (R1-O)₃CH in the presence of an acidic catalyst, characterized in that the reaction is carried out in a loop reactor.

In a preferred embodiment, the orthoformate is selected from the group consisting of trimethyl orthoformate, triethyl orthoformate, tripropyl orthoformate and tributyl orthoformate. More preferably, the orthoformate is trimethyl orthoformate.

"Continuous operation" means that both the reaction partners and the reaction products are continuously added and removed, respectively. According to the invention, the ketene gas, the orthoformate and the acidic catalyst are continuously reacted with one another in a loop reactor.

Here, the term "loop reactor" does not denote a certain design, but only the principle of operation. In the most simple case, the loop reactor consists of a circularly closed tube (loop) equipped with a circulating pump. The loop has at least one connection for withdrawing a product stream and at least two connections for feeding the starting materials.
The reaction can be carried out in a solvent or in the absence of a solvent. The acidic catalyst can be directly added into the reactor, or it can be mixed beforehand with the orthoformate and/or with the solvent. The number and the positions of the feed connections have to be chosen accordingly. Preferably, the orthoformate is firstly mixed with the acidic catalyst and optionally with the solvent, whereupon the catalyst goes into solution or just forms a suspension. The resulting mixture is then fed into the loop reactor. In a particularly preferred process variant, the reaction is carried out in the absence of a solvent.

The ketene gas can be fed into the reaction mixture by any suitable gas distribution system, for example, by using a sparger, which is optionally provided with a frit or a nozzle. Preferably, the gaseous ketene and the liquid mixture consisting of orthoformate, catalyst and, optionally, solvent is introduced using a gas-liquid ejector. A gas-liquid ejector consists of different units described as follows. The liquid flow passes through a nozzle which generates a high velocity jet of fluid, thus creating suction of the ketene and entraining it into the ejector. Advantageously, the accelerated liquid-gaseous jet collides with the wall of an adjacent mixing tube, resulting in a rapid dissipation of kinetic energy. This forms an intensive mixing shock zone, in which the high turbulences produce a fine dispersion of bubbles. The ability to generate and finally disperse very small ketene bubbles into the liquid mixture leads to a favourable gas-liquid ratio of, for example, from 0.5 to 2.0, and to a much better dispersion of ketene in the liquid. The thus obtained two-phase mixture is finally injected into the fluid phase in the reactor, leading to an optimal efficacy in the subsequent chemical reaction. In addition, this way of gas distribution allows a consistent, pressure-free flowing of ketene into the gas-liquid ejector, which is particularly desired as ketene is prone to polymerization under pressure. Optionally, a swirl device directs, orientates and stabilizes the pumped liquid flow, before the liquid flow passes through the nozzle. A type of reactor as described above is also known as BUSS Loop® reactor.

Essentially, the reaction components are fed into the loop reactor in a simultaneous and continuous manner. This means that there are no major interruptions or strong variations in the molar ratio of the reactants within the reaction mixture. The circulation in the loop ensures good or even ideal mixing. However, it is not necessary to enforce an ideal mixing.

When simultaneously charging the reactants, a product stream is withdrawn from the loop reactor, in a volume which corresponds to the volume of the reactants charged, for subjecting to the following work-up procedure. This may take place, for example, via a simple overflow pipe or by pumping-off, while the pumping-off may be controlled using a level detector.

Depending on the feed rate, efficient cooling may be required due to the highly exothermic reaction. It can be achieved by known means, like by use of a cooling jacket covering a substantial part of the tube length or by a heat exchanger of conventional construction being incorporated into the loop.

The reaction is advantageously carried out at a temperature of from -40°C to 50°C. Optionally, the reactants may be pre-cooled before feeding into the loop reactor. Preferably, the reaction temperature is of from -30°C to 30°C, more preferably of from -10°C to 10°C.

The ketene used may be essentially pure or may contain inert gases, such as nitrogen, carbon monoxide and/or carbon dioxide, which advantageously are removed from the loop reactor by means of a suitable air-relief vent in order to prevent excessive pressure buildup.

The molar ratio of orthoformate to ketene is preferably of from 0.9 to 1.2, more preferably of from 1.0 to 1.1. These values refer to the amounts charged. The ratios actually present in the reaction mixture may differ more or less from these values.

In principle, each organic solvent in which the orthoformate is sufficiently soluble and which does not react with ketene or any other component can be used as solvent. Suitable solvents are, for example, aliphatic or aromatic hydrocarbons and ethers. However, it is also possible to dispense with a solvent, provided the used orthoformate is liquid. In a preferred embodiment, trimethyl orthoformate is directly reacted with ketene, i.e. without solvent, in the presence of an acidic catalyst.

The reaction can be catalyzed by all suitable acidic catalysts. Suitable acidic catalysts are both "classic" Lewis acids and "classic" Bronsted acids, and also acidic polysilicates. Advantageously, the "classic" Lewis acids used are zinc(II) chloride, iron(III) chloride, aluminum chloride, boron trifluoride and its adducts with ethers, esters and similar compounds. A preferred adduct of boron trifluoride is the diethyl ether adduct. Preferred examples of'classic" Bronsted acids are sulfuric acid, phosphoric acid, methanesulfonic acid and benzenesulfonic acid.
Acidic polysilicates have Lewis and/or Bronsted acid properties and are therefore likewise suitable for the process according to the invention. The acidic polysilicates can also be employed in modified form or as mixtures. The formulae below are only given to illustrate the polysilicates but are not meant to be interpreted as a limitation. Suitable acidic polysilicates are, for example, amorphous polysilicates of the allophane type; chain polysilicates of the hormite type, such as "polygorskite"; two-layer polysilicates of the kaolin type, such as "kaolinite" Al₂(OH)₄[Si₂O₅] and "halloysite" Al₂(OH)₄[Si₂O₅] x 2 H₂O; three-layer polysilicates of the smectite type, such as "sauconite" Na_{0.3}Zn₃(Si,Al)₄O₁₀(OH)₂ x 4 H₂O, "saponite" (Ca,Na)_{0.3}(Mg,Fe^{[ ]})₃(Si,Al)₄O₁₀(OH)₂ x 4 H₂O,"montmorillonite" M_{0.3}(Al,Mg)₂Si₄O₁₀(OH)₂ x n H₂O,wherein M in natural montmorillonite denotes one equivalent of one or more of the cations Na⁺, K⁺, Mg²⁺ and Ca²⁺, "vermiculite" (Mg,Fe^{[ ]},Al)₃(Al,Si)₄O₁₀(OH)₂ x 4 H₂O,"nontronite" Na_{0.3}Fe₂^{III}(Si,Al)₄O₁₀(OH)₂ x 4 H₂O and "hectorite" Na_{0.3}(Mg,Li)₃Si₄O₁₀(F,OH)₂; three-layer polysilicates of the illite type; polysilicates having variable layers of the chlorite type and tectopolysilicates, such as zeolites, preferably of type Yin its H-form.
If required, the acidic polysilicates of the process according to the invention may be activated by treatment with acid and/or by treatment with a metal salt solution and/or by drying, and in the case of zeolites preferably by ion-exchange and/or by heating.
In a preferred embodiment, the catalysts used are acidic polysilicates of the smectite type and zeolites. A particularly preferred acidic polysilicate of the smectite type is montmorillonite, especially the types available under the names "montmorillonite K 10" and "montmorillonite KSF/0", which are available, for example, from the company Sud-Chemie.

The acidic catalyst is advantageously employed in the process of the invention in an amount of from 0.1% by weight to 20% by weight (based on orthoformate), preferably of from 0.5 to 10% by weight. However, the amount depends on the activity of the catalyst and the reaction temperature.

When carrying out the reaction, it has to be ensured that the water content is as low as possible, since ketene and orthoformate may react with water in an unwanted manner.

Work-up is carried out by methods commonly known in the art and essentially depends on the physical properties of the formed alkyl 3,3-dialkoxypropionate and the other components of the reaction mixture. If a solid acidic catalyst is used, this is advantageously removed by filtration and the filtrate is worked up, whereas, if a liquid acid catalyst is used, this is first neutralized in the reaction mixture. The neutralization may be carried out, for example, by adding basic alkali metal salts, such as sodium hydroxide and potassium carbonate, or by adding alkali metal alkoxides, such as sodium methoxide and potassium ethoxide, or by adding similar basic reagents, such as anhydrous ammonia. Any precipitate may then be removed by filtration, and the filtrate can subsequently be purified, if required.
In a preferred embodiment, a solid acid catalyst is used which is filtered off in a first work-up step. The residue thus obtained is then either discarded or re-used in the reaction mixture as acidic catalyst, after its purification and optional re-activation if required.

After removal of the acidic catalyst, the filtrate is worked up in a known manner, preferably by distillation, to obtain the formed alkyl 3,3-dialkoxypropionate in neat form. In a particularly preferred embodiment, the unreacted orthoformate, which usually has a lower boiling point than the desired product, is distilled off after filtration and is then re-cycled into the reaction mixture, which significantly increases the total conversion of the reaction.

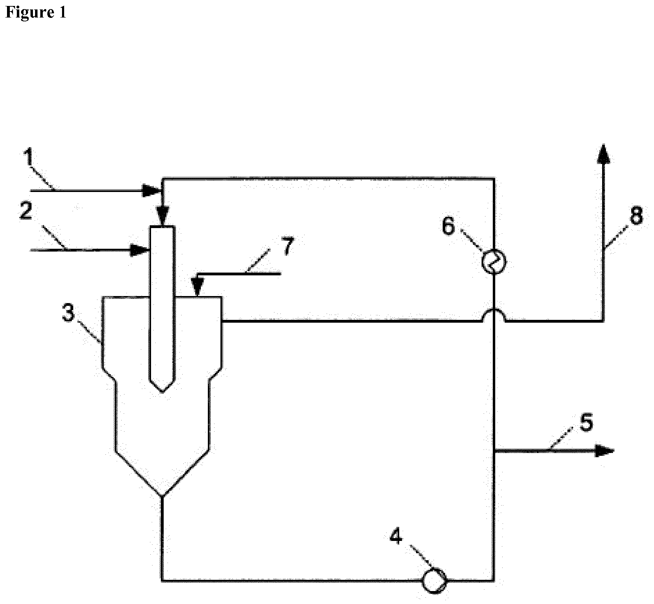

### Explanation of the Figure 1

The appended schematic Figure 1 as well as the examples serves only to illustrate the subject matter of the invention without limiting it to these disclosures.

Figure 1 shows, schematically, a device for the continuous preparation of alkyl 3,3-dialkoxypropionate. The specific meanings of the reference signs are as follows:
1. Feed of the mixture of orthoester and acidic catalyst
2. Ketene feed
3. Jet reactor
4. Circulating pump
5. Product removal
6. Heat exchanger
7. Nitrogen feed
8. Venting of inert gases

So essentially,
- compound of formula (VI) is prepared by a continuous process for preparing alkyl 3,3-dialkoxypropionates of formula (R1-O)₂CHCH₂CO₂R1, wherein R1 is defined as herein, also with all its embodiments, by reacting ketene with an orthoformate of formula (R1-O)₃CH in the presence of an acidic catalyst, characterized in that the reaction is carried out in a loop reactor.
- Preferably, the orthoformate is selected from the group consisting of trimethyl orthoformate, triethyl orthoformate, tripropyl orthoformate and tributyl orthoformate.
- Preferably, the orthoformate is firstly mixed with the acidic catalyst and only then fed into the loop reactor.
- Preferably, the loop reactor comprises a gas-liquid ejector (jet reactor).
- Preferably, the reaction is carried out at a temperature of from -40 °C to 50 °C.
- Preferably, the molar ratio of orthoformate to ketene is of from 0.9 to 1.2.
- Preferably, the process is carried out in the absence of a solvent.
- Preferably, the acidic catalyst is a Lewis acid, a Bronsted acid or an acidic polysilicate.
- Preferably, the Lewis acid is selected from the group consisting of zinc(II) chloride, iron(III) chloride, aluminum chloride, boron trifluoride and its adducts with ethers and esters.
- Preferably, the Bronsted acid is selected from the group consisting of sulfuric acid, phosphoric acid, methanesulfonic acid and benzenesulfonic acid.
- Preferably, the acidic polysilicate is selected from the group consisting of acidic, amorphous polysilicates of the allophane type; acidic, chain polysilicates of the hormite type; acidic, two-layer polysilicates of the kaolin type; acidic, three-layer polysilicates of the smectite type; acidic, three-layer polysilicates of the illite type; acidic, variable-layer polysilicates of the chlorite type; and acidic tectopolysilicates.
- Preferably, the acidic, three-layer polysilicate of the smectite type is selected from the group consisting of sauconite, saponite, montmorillonite, vermiculite, nontronite and hectorite.
- Preferably, the orthoformate is trimethyl orthoformate and the acidic catalyst is montmorillonite.
- Preferably, the acidic catalyst is employed in an amount of from 0.1% by weight to 20% by weight (based on orthoformate).

### Examples

### Protocols

### Protocol 3.1: EDFA, MDMP, sodium tert. butoxide, neat

Solid sodium tert-butoxide (38.3 g, 0.399 mol, 1.06 eq) was added portion wise over a time period of 20 min to a mixture of methyl 3,3-dimethoxypropionate (MDMP) (64.3 g, 0.434 mol, 1.16 eq) and ethyl difluoroacetate (EDFA) (46.5 g, 0.375 mol, 1 eq) at 25 to 30°C. After completion of the addition, the mixture was stirred for 60 min at 65°C. Upon cooling to room temperature, 140 g of a viscous mixture was obtained that was found to be sufficiently pure to carry forward without further purification. Quantitative ¹⁹F-NMR analysis using 1,4-difluorobenzene as internal standard of the reaction mixture revealed a 89% yield of (2-(dimethoxy-methyl)-4,4-difluoro-1-methoxy-1,3-dioxobutan-2-yl) sodium salt.

This viscous mixture was added to a mixture of acetic acid (30.2 g, 0.503 mol, 1.34 eq) and aqueous methylhydrazine 42 wt% (41.1 g, 0.375 mol, 1.00 eq) at 0 °C over a period of 1 h. After heating to 55°C, aqueous sodium hydroxide 10 wt% (255 g, 0.638 mol, 1.70 eq) was added within 1 h and the resulting mixture was stirred at 60°C for 3 h. After dilution with 266 g water, aqueous sulfuric acid 30 wt% (248 g, 0.759 mol, 2.02 eq) was added in 30 min. Then the mixture was cooled to 0 °C and stirred at 0 °C for 30 min before the crystallized product was collected by filtration, washed with 261 g water and dried at 50°C and 25 mbar to yield 40.1 g 3-(difluoromethyl)-1-methyl-1H-pyrazole-4-carboxylic acid (DFPA). The purity of the product was measured by quantitative ¹⁹F-NMR using 1,4-difluorobenzene as internal standard showing a content of 99.5 wt%, less than 0.4 wt% of the isomeric 5-(difluoromethyl)-1-methyl-1H-pyrazole-4-carboxylic acid (iso-DFP) and a fluorine purity of 99.6 mol% corresponding to a yield of 60.7 %.

### Protocol 3.3: EDFA, MDMP, potassium tert. butoxide, neat

Solid potassium tert. butoxide (44.7 g, 0.398 mol, 1.06 eq) was added portion wise over a time period of 15 min to a mixture of methyl 3,3-dimethoxypropionate (MDMP) (64.3 g, 0.434 mol, 1.16 eq) and ethyl difluoroacetate (EDFA) (46.5 g, 0.375 mol, 1 eq) at 25 to 30°C. After completion of the addition, the mixture was stirred for 2 h at 65°C. This mixture analyzed by quantitative ¹⁹F-NMR analysis using 1,4-difluorobenzene as internal standard revealing a yield of 82% of (2-(dimethoxy-methyl)-4,4-difluoro-1-methoxy-1,3-dioxobutan-2-yl) potassium salt.

### Protocol 3.5: EDFA, MDMP, Sodium tert. butoxide, neat

Solid sodium tert. butoxide (51.0 g, 0.531 mol, 1.06 eq) was added portion wise over a time period of 10 min to a mixture of methyl 3,3-dimethoxypropionate (MDMP) (85.2 g, 0.575 mol, 1.15 eq) and ethyl difluoroacetate (EDFA) (62.0 g, 0.500 mol, 1.00 eq) at 25 to 30 °C. After completion of the addition, the mixture was stirred for 6 h at 65 °C. The reaction mixture was analyzed by quantitative ¹⁹F-NMR analysis using 1,4-difluorobenzene as internal standard revealing a yield of 90 % of (2-(dimethoxy-methyl)-4,4-difluoro-1-methoxy-1,3-dioxobutan-2-yl) sodium salt.

The reaction mixture was added to a mixture of acetic acid (HOAc) (40.2 g, 0.67 mol, 1.34 eq) and aqueous methyl hydrazine (MeH) 42 wt% (54.9 g, 0.500 mol, 1.00 eq) at 15 °C. The resulting reaction mixture was then heated to 65 °C for 2 h. Analysis of this reaction mixture by quantitative ¹⁹F-NMR using 1,4-difluorobenzene as internal standard showed the formation of methyl 3-(difluoromethyl)-1-methyl-1H-pyrazole-4-carboxylate (DFP-ester) in 81 % yield.

### Protocol 4: Acid screening

Solid sodium tert-butoxide (253 g, 2.63 mol, 1.05 eq) was added portion wise over a time period of 40 min to a mixture of methyl 3,3-dimethoxypropionate (MDMP) (428 g, 2.89 mol, 1.16 eq) and ethyl difluoroacetate (EDFA) (310 g, 2.50 mol, 1.00 eq) at 25 to 30 °C. After completion of the addition, the mixture was stirred for 60 min at 65 °C. After cooling to room temperature, 983 g of a viscous mixture was obtained that was found to be sufficiently pure to carry forward without further purification. Quantitative ¹⁹F-NMR analysis using 1,4-difluorobenzene as internal standard of the reaction mixture revealed a yield of 88 to 90 % of (2-(dimethoxy-methyl)-4,4-difluoro-1-methoxy-1,3-dioxobutan-2-yl) sodium salt of several repetitions of this protocol.

### Protocol 4.2: Formic acid

In order to screen the influence of various acids, 209 g the reaction mixture, prepared according to protocol 4 (corresponding to 0.532 mol EDFA usage = 1.00 eq), was added to a mixture of formic acid (32.8 g, 0.712 mol, 1.34 eq) and aqueous methylhydrazine 42 wt% (57.3 g, 0.522 mol, 0.98 eq) at 65 °C over a period of 1.5 h. The resulting reaction mixture was stirred at 65 °C for 1 h and analyzed by quantitative ¹⁹F-NMR analysis using 1,4-difluorobenzene as internal standard to measure the yield of the reaction (see values in Table 1).

### Protocol 4.3: Citric acid

In order to screen the influence of various acids, 209 g the reaction mixture, prepared according to protocol 4 (corresponding to 0.532 mol EDFA usage = 1.00 eq), was added to a mixture of citric acid (44.8 g, 0.233 mol, 0.439 eq) and aqueous methylhydrazine 42 wt% (57.3 g, 0.522 mol, 0.98 eq) at 65 °C over a period of 1.5. The resulting reaction mixture was stirred at 65°C for 1 h before it was diluted with 250 g water. The organic phase was analyzed by quantitative ¹⁹F-NMR analysis using 1,4-difluorobenzene as internal standard to measure the yield of the reaction (see values in Table 1).

### Protocol 4.5: Lactic acid

In order to screen the influence of various acids, 165 g the reaction mixture, prepared according to protocol 4 (corresponding to 0.420 mol EDFA usage = 1.00 eq), was added to a mixture of lactic acid 85% (59.6 g, 0.562 mol, 1.34 eq) and aqueous methylhydrazine 42% (45.2 g, 0.412 mol, 0.98 eq) at 65°C over a period of 1.5h. The resulting reaction mixture was stirred at 65 °C for 1 h and analyzed by quantitative ¹⁹F-NMR analysis using 1,4-difluorobenzene as internal standard to measure the yield of the reaction (see values in Table 1).

### Protocol 5: Ionic exchange screen

Solid sodium tert-butoxide (253 g, 2.63 mol, 1.05 eq) was added portion wise over a time period of 40 min to a mixture of methyl 3,3-dimethoxypropionate (MDMP) (428 g, 2.89 mol, 1.16 eq) and ethyl difluoroacetate (EDFA) (310 g, 2.50 mol, 1.00 eq) at 25 to 30 °C. After completion of the addition, the mixture was stirred for 60 min at 65 °C. After cooling to room temperature, 983 g of a viscous mixture was obtained that was found to be sufficiently pure to carry forward without further purification. Quantitative ¹⁹F-NMR analysis using 1,4-difluorobenzene as internal standard of the reaction mixture revealed a yield of 88 to 90 % of (2-(dimethoxy-methyl)-4,4-difluoro-1-methoxy-1,3-dioxobutan-2-yl) sodium salt of several repetitions of this protocol.

110 g this reaction mixture (corresponding to 0.393 mol EDFA usage = 1.00 eq) was dissolved in 66 g solvent (see Table 1) and added to a mixture of 100 ml Dowex MAC-3 H ion exchange (The Dow Chemical Company, Midland, Michigan, US) or 37.5 g Amberlite CG50 ion exchange (The Dow Chemical Company, Midland, Michigan, US), aqueous methylhydrazine 42 wt% (for eq see Table 1) and 44 g solvent (see Table 1) at 15 °C over a period of 1 h. The resulting reaction mixture was stirred at 65 °C for 1h and analyzed by quantitative ¹⁹F-NMR analysis using 1,4-difluorobenzene as internal standard to measure the yield of the reaction (see Table 1)

### Protocol 6: Solvent screening

Solid sodium tert-butoxide (253 g, 2.63 mol, 1.05 eq) was added portion wise over a time period of 40 min to a mixture of methyl 3,3-dimethoxypropionate (MDMP) (428 g, 2.89 mol, 1.16 eq) and ethyl difluoroacetate (EDFA) (310 g, 2.50 mol, 1.00 eq) at 25 to 30 °C. After completion of the addition, the mixture was stirred for 60 min at 65 °C. After cooling to room temperature, 983 g of a viscous mixture was obtained that was found to be sufficiently pure to carry forward without further purification. Quantitative ¹⁹F-NMR analysis using 1,4-difluorobenzene as internal standard of the reaction mixture revealed a yield of 88 to 90 % of (2-(dimethoxy-methyl)-4,4-difluoro-1-methoxy-1,3-dioxobutan-2-yl) sodium salt of several repetitions of this protocol.

In order to screen the influence of various solvents, 110 g this reaction mixture (corresponding to 0.393 mol EDFA usage = 1.00 eq) were dissolved in 77 g solvent (see Table 1) and added to a mixture of acid (see eq in Table 1), aqueous methylhydrazine 42 wt% (see eq in Table 1) and 33 g solvent (see Table 1) at 15 °C over a period of 1 h. The resulting reaction mixture was heated to 65 °C for 2 h and analyzed by quantitative ¹⁹F-NMR analysis using 1,4-difluorobenzene as internal standard to measure the yield of the reaction (see Table 1).

### Protocol 7: EDFA, 1.15 eq. MDMP, 1.05 eq. sodium tert. butoxide, 1 eq MeH, 1.34 eq AcOH

Solid sodium tert-butoxide (253 g, 2.63 mol, 1.05 eq) was added portion wise over a time period of 40 min to a mixture of methyl 3,3-dimethoxypropionate (MDMP) (428 g, 2.89 mol, 1.16 eq) and ethyl difluoroacetate (EDFA) (310 g, 2.50 mol, 1.00 eq) at 25 to 30 °C. After completion of the addition, the mixture was stirred for 2 h min at 65 °C. After cooling to room temperature, 979 g of a viscous mixture was obtained that was found to be sufficiently pure to carry forward without further purification. Quantitative ¹⁹F-NMR analysis using 1,4-difluorobenzene as internal standard of the reaction mixture revealed a yield of 89 % of (2-(dimethoxy-methyl)-4,4-difluoro-1-methoxy-1,3-dioxobutan-2-yl) sodium salt.

A 154 g aliquote of this viscous mixture was dissolved in 108 g 2-methyltetrahydrofuran (MeTHF) and added to a mixture of acetic acid (31.5 g, 0.52 mol, 1.33 eq), aqueous methylhydrazine 42 wt% (42.2 g, 0.385 mol, 0.98 eq) and 46.2 g 2-methyltetryhydrofuren (MeTHF) at 45°C over a time period of 1.5 h. The resulting solution was stirred for an 1 h at 65 °C to afford a reaction solution containing 64.2 g methyl 3-(difluoromethyl)-1-methyl-1H-pyrazole-4-carboxylate (DFP-ester). This corresponds to a yield of 80 % according to quantitative ¹⁹F-NMR using 1,4-difluorobenzene as internal standard.
Furthermore, the isomeric methyl 5-(difluoromethyl)-1-methyl-1H-pyrazole-4-carboxylate (isoDFP-ester) was formed in 5 %. This reaction solution was then mixed with aqueous sodium hydroxide 10 wt% (240 g, 0.600 mol, 1.53 eq) and stirred for 4 h at 55 °C. Then the organic phase was separated and the aqueous phase was mixed with aqueous sulfuric acid 30 wt% (133 g, 0.407 mol, 1.04 eq). After cooling to 0 °C, a precipitated product was isolated by filtration, washed with 300 g water and dried at 50 °C and 25 mbar to yield 44.3 g DFPA. The purity of the product was measured by quantitative ¹⁹F-NMR using 1,4-difluorobenzene as internal standard showing a content of 99.1 wt%, 0.1 wt% of the isomeric 5-(difluoromethyl)-1-methyl-1H-pyrazole-4-carboxylic acid (iso-DFP) and a fluorine purity of 99.9 mol% corresponding to a yield of 64.0 %.

In Tables 1 and 2 the examples are summarized, in case of discrepancies between the content of the Tables 1 and 2 and the content of the Protocols, then the content of the Protocols prevail.

| **Table 1** | | | | | | | |
|---|---|---|---|---|---|---|---|
| Example | Protocol | EDFA (= 1eq) | Base | | MDMP | Solvent | DF-DMPNa (in reaction mixture) |
| | | mol | | eq | eq | | yield |
| 3 | 3.1 | 0.375 | NaOtBu | 1.06 | 1.16 | Neat | 89% |
| 5 | 3.3 | 0.375 | KOtBu | 1.06 | 1.16 | Neat | 82% |
| 7 | 3.5 | 0.5 | NaOtBu | 1.06 | 1.16 | Neat | 90% |
| 9 | 4.2 | 2.5 | NaOtBu | 1.05 | 1.16 | Neat | 89% |
| 10 | 4.3 | 2.5 | NaOtBu | 1.05 | 1.16 | Neat | 90% |
| 12 | 4.5 | 2.5 | NaOtBu | 1.05 | 1.16 | Neat | 90% |
| 13 | 5 | 2.5 | NaOtBu | 1.05 | 1.16 | 2-MeTHF | 89% |
| 14 | 5 | 2.5 | NaOtBu | 1.05 | 1.16 | 2-MeTHF | 89% |
| 15 | 6 | 2.5 | NaOtBu | 1.06 | 1.16 | MTBE | 90% |
| 16 | 6 | 2.5 | NaOtBu | 1.06 | 1.16 | Toluene | 90% |
| 17 | 6 | 2.5 | NaOtBu | 1.06 | 1.16 | n-Butylacetate | 90% |
| 18 | 6 | 2.5 | NaOtBu | 1.06 | 1.16 | Methanol | 90% |
| 19 | 6 | 2.5 | NaOtBu | 1.06 | 1.16 | Diisopropylether | 88% |
| 21 | 6 | 2.5 | NaOtBu | 1.06 | 1.16 | 2-MeTHF | 89% |
| 22 | 7 | 2.5 | NaOtBu | 1.06 | 1.16 | 2-MeTHF | 89% |

| **Table 2** | | | | | | |
|---|---|---|---|---|---|---|
| Example | Protocol | MeH | Acid | DFP-ester / isoDFP-ester (in reaction mixture) overall | DFP overall | DFP : isoDFP |
| | | eq | | yield | yield | Ratio |
| 3 | 3.1 | 1 | AcOH | | 60.7% | 100 : 0.4 |
| 5 | 3.3 | - | - | - | - | - |
| 7 | 3.5 | 1 | AcOH | 81% / 8% | - | - |
| 9 | 4.2 | 1 | Formic acid | 82% / 13% | - | - |
| 10 | 4.3 | 1 | Citric acid | 85%/11% | - | - |
| 12 | 4.5 | 1 | Lactic acid 85% | 85% / 14% | - | - |
| 13 | 5 | 1 | Dowex Mac-3 | 86% / 8% | - | - |
| 14 | 5 | 1 | Amberlite CG50 | 90% / 7% | - | - |
| 15 | 6 | 1 | AcOH | 88% / 9% | - | - |
| 16 | 6 | 1 | AcOH | 86% / 12% | - | - |
| 17 | 6 | 1 | AcOH | 88% / 9% | - | - |
| 18 | 6 | 1 | AcOH | 83% / 10% | - | - |
| 19 | 6 | 1 | AcOH | 84%/10% | - | - |
| 21 | 6 | 1 | AcOH | 80% / 5% | - | - |
| 22 | 7 | 1 | AcOH | 80% / 5% | 64% | 100 : 0.2 |

### Example 31: Preparation of methyl3,3-dimethoxypropionate

Simultaneously, but separately, 150 kg/h (1.413 kmol/h) oftrimethyl orthoformate (Fluka), comprising 1.5% by weight of montmorillonite K10 (Siid-Chemie), and 84 kg/h of ketene (ketene content about 70%, remainder inert gases, such as N2, CO and C02, i.e. neat ketene about 59 kg/h, that is about 1.4 kmol/h) were fed into a 620 L jet reactor (see figure 1), which had been inertized and cooled to an internal temperature of 0°C. Under an atmosphere of nitrogen, the reaction mixture was kept at a temperature of about 0 °C and circulated in the loop via a circulating pump. Corresponding to the amount of starting materials added, and also continuously, a corresponding part of the reaction mixture flowed over into a collecting tank.

After filtration, the purity of the filtrate was determined by GC as 80% methyl 3,3-dimethoxypropionate, 8% unreacted trimethyl orthoformate, 4% methyl3-methoxyprop-2-enoate and 4% methyl acetate.

By virtue of its low boiling point, it was easy to remove trimethyl orthoformate by distillation. The recovered starting material was subsequently re-cycled into the reaction mixture.

The yield of methyl 3,3-dimethoxypropionate was 82% (based on conversion).

## Claims

1. A method for the preparation of compound of formula (I); 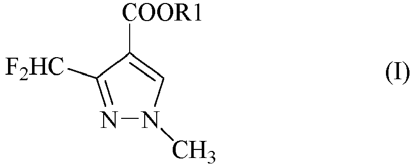 the method comprises two steps, a step STEP1 and a step STEP2;
STEP1 comprises a reaction REAC1 of compound of formula (VII) and compound of formula (VI) 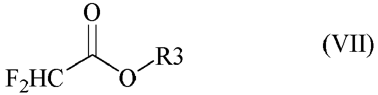 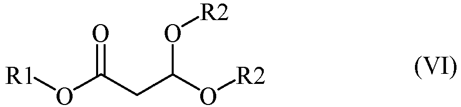 in the presence of a base BAS1, BAS1 is compound of formula (VIII); 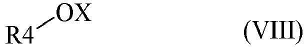 R1, R2, R3 and R4 are identical or different and independently from each other C₁₋₄ alkyl; X is Na or K;
STEP2 comprises a reaction REAC2 of the reaction product of REAC1 with methyl hydrazine in the presence of an acid ACID2;
ACID2 is selected from the group consisting of AcOH, H₂SO₄, HCOOH, citric acid, benzoic acid, lactic acid and acidic ion exchange resin.

2. Method according to claim 1, wherein R1, R2, R3 and R4 are identical or different and independently from each other selected from the group consisting of methyl, ethyl, propyl, and butyl.

3. Method according to claim 1 or 2, wherein R1 and R2 are identical.

4. Method according to one or more of claims 1 to 3, wherein X is Na.

5. Method according to one or more of claims 1 to 4, wherein REAC1 is done neat or in the presence of a solvent SOLV1;
SOLV1 is selected from the group consisting of methanol, ethanol, n-propanol, isopropanol, n-butanol, iso-butanol, sec-butanol, tert-butanol, THF, 2-MeTHF and MTBE, toluene, n-butyl acetate, and diisopropylether.

6. Method according to one or more of claims 1 to 5, wherein the acidic ion exchange resin is a polymeric sulfonic acid resin.

7. Method according to one or more of claims 1 to 6, wherein ACID2 is selected from the group consisting of AcOH, H₂SO₄, HCOOH, citric acid, benzoic acid, and lactic acid.

8. Method according to one or more of claims 1 to 7, wherein REAC2 is done neat or in the presence of a solvent SOLV2;
SOLV2 is selected from the group consisting of methanol, ethanol, n-propanol, isopropanol, n-butanol, iso-butanol, sec-butanol, tert-butanol, THF, 2-MeTHF, MTBE, toluene, n-butyl acetate, and diisopropyl ether.

9. Method according to one or more of claims 1 to 8, wherein REAC2 is done in the presence of water.

10. A method for the preparation of compound of formula (3) or the sodium or potassium salt of compound of formula (3); 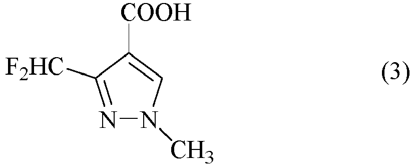 the method comprises the two steps STEP1, STEP2 and a third step STEP3;
with STEP1 and STEP2 as described in claim 1;
in STEP3 compound of formula (I) obtained from STEP2 is saponified.

11. Method according to claim 10, wherein the saponification in STEP3 is done in the presence of water.

## Patentansprüche

1. Verfahren zur Herstellung einer Verbindung der Formel (I); 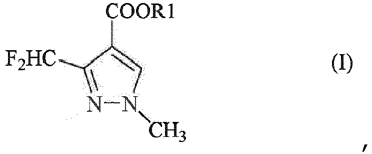 wobei das Verfahren zwei Schritte umfasst, einen Schritt STEP1 und einen Schritt STEP2;
STEP1 eine Umsetzung REAC1 einer Verbindung der Formel (VII) und einer Verbindung der Formel (VI) 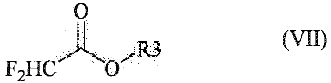 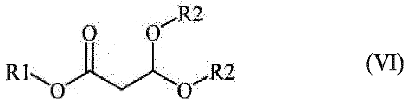 in Gegenwart einer Base BAS1 umfasst, BAS1 eine Verbindung der Formel (VIII) ist; 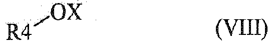 R1, R2, R3 und R4 gleich oder verschieden sind und unabhängig voneinander für C₁₋₄-Alkyl stehen;
X für Na oder K steht;
STEP2 eine Umsetzung REAC2 des Reaktionsprodukts von REAC1 mit Methylhydrazin in Gegenwart einer Säure ACID2 umfasst;
ACID2 aus der Gruppe bestehend aus AcOH, H₂SO₄, HCOOH, Citronensäure, Benzoesäure, Milchsäure und saurem Ionenaustauscherharz ausgewählt wird.

2. Verfahren nach Anspruch 1, wobei R1, R2, R3 und R4 gleich oder verschieden sind und unabhängig voneinander aus der Gruppe bestehend aus Methyl, Ethyl, Propyl und Butyl ausgewählt sind.

3. Verfahren nach Anspruch 1 oder 2, wobei R1 und R2 gleich sind.

4. Verfahren nach einem oder mehreren der Ansprüche 1 bis 3, wobei X für Na steht.

5. Verfahren nach einem oder mehreren der Ansprüche 1 bis 4, wobei REAC1 in Substanz oder in Gegenwart eines Lösungsmittels SOLV1 durchgeführt wird; SOLV1 aus der Gruppe bestehend aus Methanol, Ethanol, n-Propanol, Isopropanol, n-Butanol, Isobutanol, sec-Butanol, tert-Butanol, THF, 2-MeTHF und MTBE, Toluol, Essigsäure-n-butylester und Diisopropylether ausgewählt wird.

6. Verfahren nach einem oder mehreren der Ansprüche 1 bis 5, wobei es sich bei dem sauren Ionenaustauscherharz um ein polymeres Sulfonsäureharz handelt.

7. Verfahren nach einem oder mehreren der Ansprüche 1 bis 6, wobei ACID2 aus der Gruppe bestehend aus AcOH, H₂SO₄, HCOOH, Citronensäure, Benzoesäure und Milchsäure ausgewählt wird.

8. Verfahren nach einem oder mehreren der Ansprüche 1 bis 7, wobei REAC2 in Substanz oder in Gegenwart eines Lösungsmittels SOLV2 durchgeführt wird; SOLV2 aus der Gruppe bestehend aus Methanol, Ethanol, n-Propanol, Isopropanol, n-Butanol, Isobutanol, sec-Butanol, tert-Butanol, THF, 2-MeTHF und MTBE, Toluol, Essigsäure-n-butylester und Diisopropylether ausgewählt wird.

9. Verfahren nach einem oder mehreren der Ansprüche 1 bis 8, wobei REAC2 in Gegenwart von Wasser durchgeführt wird.

10. Verfahren zur Herstellung der Verbindung der Formel (3) oder des Natrium- oder Kaliumsalzes der Verbindung der Formel (3); 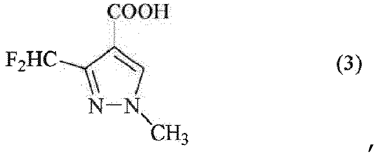 wobei das Verfahren die beiden Schritte STEP1, STEP2 und einen dritten Schritt STEP3 umfasst; wobei STEP1 und STEP2 wie in Anspruch 1 beschrieben sind;
in STEP3 die aus STEP2 erhaltene Verbindung der Formel (I) verseift wird.

11. Verfahren nach Anspruch 10, wobei die Verseifung in STEP3 in Gegenwart von Wasser durchgeführt wird.

## Revendications

1. Procédé pour la préparation d'un composé de formule (I) ; 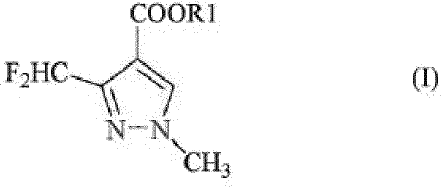 le procédé comprenant deux étapes, une étape STEP1 et une étape STEP2 ;
STEP1 comprenant une réaction REAC1 d'un composé de formule (VII) et d'un composé de formule (VI) 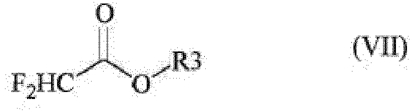 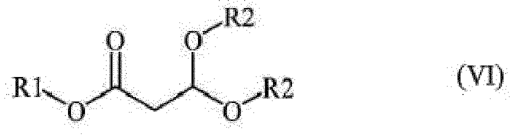 en la présence d'une base BAS1, BAS1 étant un composé de formule (VIII) ; 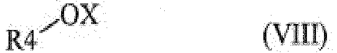 R1, R2, R3 et R4 étant identiques ou différents et étant indépendamment les uns des autres C₁₋₄ alkyle;
X étant Na ou K ;
STEP2 comprenant une réaction REAC2 du produit de réaction de REAC1 avec de la méthylhydrazine en la présence d'un acide ACID2 ;
ACID2 étant choisi dans le groupe constitué par AcOH, H₂SO₄, HCOOH, l'acide citrique, l'acide benzoïque, l'acide lactique et une résine acide d'échange d'ions.

2. Procédé selon la revendication 1, R1, R2, R3 et R4 étant identiques ou différents et étant indépendamment les uns des autres choisis dans le groupe constitué par méthyle, éthyle, propyle et butyle.

3. Procédé selon la revendication 1 ou 2, R1 et R2 étant identiques.

4. Procédé selon l'une ou plusieurs des revendications 1 à 3, X étant Na.

5. Procédé selon l'une ou plusieurs des revendications 1 à 4, REAC1 étant réalisée à l'état pur ou en la présence d'un solvant SOLV1 ;
SOLV1 étant choisi dans le groupe constitué par le méthanol, l'éthanol, le n-propanol, l'isopropanol, le n-butanol, l'iso-butanol, le sec-butanol, le tert-butanol, THF, le 2-MeTHF et le MTBE, le toluène, l'acétate de n-butyle, et le diisopropyléther.

6. Procédé selon l'une ou plusieurs des revendications 1 à 5, la résine acide d'échange d'ions étant une résine d'acide sulfonique polymérique.

7. Procédé selon l'une ou plusieurs des revendications 1 à 6, ACID2 étant choisi dans le groupe constitué par AcOH, H₂SO₄, HCOOH, l'acide citrique, l'acide benzoïque, et l'acide lactique.

8. Procédé selon l'une ou plusieurs des revendications 1 à 7, REAC2 étant réalisée à l'état pur ou en la présence d'un solvant SOLV2 ;
SOLV2 étant choisi dans le groupe constitué par le méthanol, l'éthanol, le n-propanol, l'isopropanol, le n-butanol, l'iso-butanol, le sec-butanol, le tert-butanol, THF, le 2-MeTHF, le MTBE, le toluène, l'acétate de n-butyle, et le diisopropyléther.

9. Procédé selon l'une ou plusieurs des revendications 1 à 8, REAC2 étant réalisée en la présence d'eau.

10. Procédé pour la préparation d'un composé de formule (3) ou du sel de sodium ou de potassium du composé de formule (3) ; 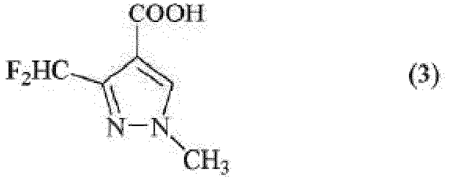 le procédé comprenant les deux étapes STEP1, STEP2 et une troisième étape STEP3 ;
STEP1 et STEP2 étant telles que décrites dans la revendication 1 ;
dans STEP3, le composé de formule (I) obtenu de l'étape STEP2 étant saponifié.

11. Procédé selon la revendication 10, la saponification dans l'étape STEP3 étant réalisée en la présence d'eau.
